# FASCICULE DE BREVET EUROPEEN

(11) **EP 2 344 206 B1**
(45) Date de publication et mention de la délivrance du brevet: **13.04.2016**
(21) Numéro de dépôt: 09740172.3
(22) Date de dépôt: 14.09.2009
(51) Int. Cl.: A61L 9/20, B01D 53/85, B01D 53/86, B01J 19/12, B01J 21/18, B01J 27/20, B01J 35/00, B01J 35/04, C02F 1/32, F24F 3/16

(54) **PHOTOREACTEUR COMPORTANT UN PHOTOCATALYSEUR A BASE DE MOUSSES TRIDIMENSIONNELLES STRUCTUREES EN CARBONE**
PHOTOREAKTOR ENTHALTEND EINEN PHOTOKATALYSATOR BESTEHEND AUS DREIDIMENSIONAL STRUKTURIERTEN KOHLENSTOFFSCHÄUMEN
PHOTOREACTOR COMPRISING A PHOTOCATALYST BASED ON THREE-DIMENSIONAL STRUCTURED CARBON FOAMS

(30) Priorité: 12.09.2008 FR 0805023
(43) Date de publication de la demande: 20.07.2011
(73) Titulaire: Centre National de la Recherche Scientifique, 75794 Paris Cedex 16 (FR); Université de Strasbourg, 67081 Strasbourg Cedex (FR)
(72) Inventeur: PHAM-HUU, Cuong, F-67700 Saverne (FR); KELLER, Nicolas, F-67200 Strasbourg (FR); LEDOUX, Marc-Jacques, F-67000 Strasbourg (FR); KELLER-SPITZER, Valérie, F-67203 Oberschaeffolsheim (FR); BEGIN, Dominique, F-67204 Achenheim (FR); BERNHARDT, Pierre, F-67190 Heiligenberg (FR); JOSSET, Sébastien, F-67000 Strasbourg (FR); HAJESMAILI, Shabnam, F-67000 Strasbourg (FR); ROMERO, Thierry, F-67200 Strasbourg (FR); WURTZ, Nathanaëlle, 57150 Creutzwald (FR); DOSS, Nizar, 67200 Strasbourg (FR)
(74) Mandataire: Schmidt, Martin Peter
(86) Numéro de dépôt international: PCT/FR2009/001092
(87) Numéro de publication internationale: WO 2010/029234

(56) Documents cités:
- EP-A- 1 405 718
- EP-A1- 1 656 985
- WO-A2-2009/098393
- JP-A- 2002 060 692
- JP-A- 2003 192 817
- JP-A- 2004 148 305
- JP-A- 2004 315 722
- JP-A- 2006 224 026
- KR-A- 20030 075 888
- US-A1- 2008 178 738
- INAGAKI M ET AL: "Carbon foams prepared from polyimide using urethane foam template" CARBON, ELSEVIER, OXFORD, GB, vol. 42, no. 3, 1 janvier 2004 (2004-01-01), pages 497-502, XP004488607 ISSN: 0008-6223
- XIE Y ET AL: "The effects of synthesis temperature on the structure and visible-light-induced catalytic activity of F-N-codoped and S-N-codoped titania" JOURNAL OF MOLECULAR CATALYSIS. A, CHEMICAL, ELSEVIER, AMSTERDAM, NL, vol. 285, no. 1-2, 18 avril 2008 (2008-04-18), pages 142-149, XP022656055 ISSN: 1381-1169 [extrait le 2008-01-31]

## Description

### Domaine technique de l'invention

L'invention concerne un photoréacteur comportant des photocatalyseurs à base de mousses tridimensionnelles structurées, notamment à base de mousses alvéolaires de carbone, ainsi que des procédés de son utilisation pour catalyser des réactions chimiques ou la destruction de microbes, notamment dans le but de décontaminer des effluents liquides ou gazeux.

### Etat de la technique

La photocatalyse permet des réactions chimiques intéressantes, stimulées par la lumière en présence d'un photocatalyseur. Un des problèmes que pose cette approche est la conception du réacteur, qui doit permettre une grande surface d'échange entre le milieu réactionnel et le catalyseur, une faible perte de charge dans le cas des réacteurs continus, et une forte transmission de lumière. Un problème est de disposer d'une grande surface d'échange illuminée entre le photocatalyseur et le milieu réactionnel.

Quand on utilise des substrats à base de papier ou de matière non tissée par exemple, on ne peut pas travailler en flux traversant avec des épaisseurs importantes de substrat, car la perte de charge serait trop importante. Pour avoir un contact suffisant entre le milieu réactionnel et la phase photocatalytiquement active, soit on utilise des surfaces de papier importantes pour obtenir un effet catalytique suffisant (voir le brevet US 6,906,001 (Ahlstrom Research and Services) qui propose d'appliquer le photocatalyseur sur les panneaux de faux plafonds de pièces d'habitation.), soit, et notamment pour des applications en génie chimique, on doit travailler en flux léchant.

On a donc recherché des substrats poreux ou structurés pour augmenter leur surface.

A titre d'exemple, la demande de brevet WO 03/037509 (SICAT, CNRS et Université Louis Pasteur) décrit un procédé de purification d'effluents gazeux à l'aide d'un photocatalyseur poreux comprenant du SiC, du TiO₂ et du WO₃.

La demande de brevet WO 2006/061518 (CNRS et Université Louis Pasteur) décrit un procédé d'inactivation d'agents biologiques dispersés en milieu gazeux par un semi-conducteur photo-activé à base de TiO₂ déposé sur la surface interne d'un réacteur ; ce réacteur présente à l'intérieur des saillies afin d'augmenter sa surface interne. L'article « Influence of the geometry of a monolithic support on the efficiency of photocatalyst for air cleaning » par M. Furman et al. (Chemical Engineering Science vol. 62, p. 5312-5316 (2007)) présente une étude-modèle d'un réacteur photocatalytique à support poreux. Le support en résine époxy a été préparé par stéréolithographie, et du TiO₂ a été déposé comme photocatalyseur.

On connait l'utilisation de photocatalyseurs se présentant sous la forme d'une mousse, ou déposés sur un support se présentant sous la forme d'une mousse. On a utilisé notamment des photocatalyseurs à base d'une mousse de TiO₂, ou des photocatalyseurs de TiO₂ déposé sur un support sous forme de mousse, notamment le nickel et l'alumine. Les articles « Préparation of titania foams having an open cellular structure and their application to photocatalysis » par A. Yamamoto et H. Imai (Journal of Catalysis, vol. 226, p. 462-465 (2004)) et « The design and photoreaction kinetic modeling of a gas-phase titania foam packed bed reactor » par A.O. Ibhadon (Chemical Engineering Journal vol. 133, p. 317-323 (2007)) décrivent la préparation d'une mousse de TiO₂ et son utilisation photocatalytique pour la dégradation de l'acétaldéhyde et du benzène ou toluène, respectivement.
L'article « Design considérations of photocatalytic oxidation reactors using TiO2-coating foam nickels for degrading indoor gaseous formaldehyde" par L. Yang et al. (Catalysis Today vol. 126, p. 359-368 (2007)) décrit un réacteur comportant une couche mince de TiO₂, d'une épaisseur optimale de 80 nm (pour une longueur d'onde excitatrice de 254 nm), déposée sur une mousse de nickel ; l'épaisseur de la mousse de nickel est limitée à environ 2 mm, à cause de son absorption optique.
L'article « Three-phase photocatalysis using suspended titania and titania supported on a reticulated foam monolith for water purification » par I.J. Ochuma et al. (Catalysis Today, vol. 128, p. 100-107 (2007)) décrit l'utilisation d'un photocayalyseur à base de TiO₂, déposé par vaporisation d'une suspension de TiO₂ sur une mousse d'alumine, pour la dégradation du DBU (1,8-diazabicyclo[5,4,0]undec-7-ene contenu dans un effluent aqueux.
L'article « Potential of Silver Nanoparticle-Coated Polyurethane Foam as an Antibacterial Water Filter » par Prashant Jain et T. Pradeep, paru le 5 avril 2005 dans le revue Biotechnology and Bioengineering, vol. 90 (1), p. 59 - 62, décrit la fixation de nanoparticules d'argent sur un support de mousse de polyuréthane.
L'article « Carbon foams prepared from polyimide using polyurethane foam template » par Inagaki et al., paru dans la revue Carbon 42, p. 497-502 (2004), décrit le dépôt d'anatase sur des mousses de carbone avec des macropores de petite taille, d'un diamètre d'environ 50 µm à 500 µm. Les données sur l'efficacité d'un tel catalyseur sont rudimentaires. Le document US 2008/0178738 (Foamex L.P.) décrit le dépôt d'anatase sur une mousse de polyuréthane de porosité inconnue.

On connaît également les mousses de β-SiC, qui peuvent servir comme support de catalyseur. La demande de brevet WO 2007/000506 (TOTAL S.A.) décrit un procédé de transformation de monoxyde de carbone et d'hydrogène en hydrocarbures selon la réaction de Fischer-Tropsch, dans lequel une mousse alvéolaire de β-SiC est utilisée comme support de catalyseur.

On connaît également les mousses métalliques, qui peuvent être utilisées comme support de catalyseur, mais qui, outre leur prix et poids élevés, peuvent poser des problèmes de corrosion.

Le problème que la présente invention cherche à résoudre est de présenter un nouveau photoréacteur comportant un photocatalyseur pour catalyse hétérogène, à faible perte de charge et grande surface spécifique développée, et présentant une bonne inertie chimique.

### Figures

Les figures 1 à 5 concernent des modes de réalisation de l'invention.
La figure 1 montre la micrographie obtenue par microscopie électronique à balayage d'un dépôt de polysiloxane sur une mousse alvéolaire de polyuréthane par le dépôt d'un sol-gel aqueux de silice modifiée, selon un mode de réalisation de l'invention.
La figure 2 montre des micrographies obtenues par microscopie électronique à balayage de dépôts de TiO₂ obtenus par le dépôt successif de couches individuelles sur mousse de carbone : dépôt de 5 couches (figure 2A), 10 couches (figure 2B), 15 couches (fig. 2C).
Les figures 3 à 5 illustrent des modes de réalisation d'un réacteur cylindrique selon l'invention. Dans ce réacteur, le support de catalyseur est assemblé à partir d'anneaux cylindriques de mousse alvéolaire

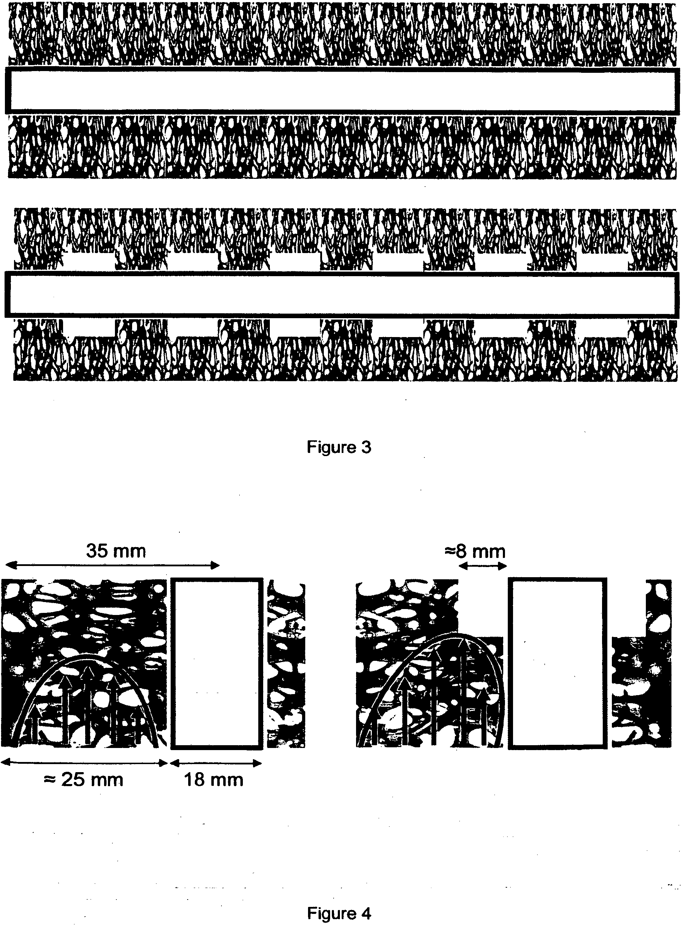

La figure 3 montre de manière schématique l'intérieur d'un réacteur tubulaire comportant une mousse alvéolaire assemblée à partir d'anneaux cylindriques identiques à titre comparatif (a) ou de forme alternativement différente (b) selon l'invention.
La figure 4 montre les cotes des anneaux cylindriques de ces réacteurs, ainsi qu'un profil de vitesse de particules.

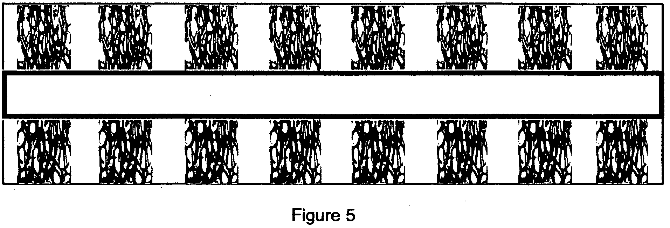

La figure 5 montre un exemple d'un réacteur présentant une configuration structurée en alternance avec 8 éléments de mousse équidistants.

### Objets de l'invention

Selon l'invention, le problème est résolu par un photoréacteur comportant un photocatalyseur comportant une mousse alvéolaire en carbone et une phase photocatalytiquement active, déposée directement sur ladite mousse alvéolaire ou sur une phase intermédiaire déposée sur ladite mousse alvéolaire. La taille moyenne des alvéoles comprise entre 2500 µm et 5000 µm, et de préférence entre 3000 µm et 5000 µm. Sa densité est avantageusement comprise entre 0,1 g/cm³ et 0,4 g/cm³. Le photocatalyseur utilisé dans le cadre de l'invention peut présenter dans le spectre visible entre 400 nm et 700 nm une transmission optique globale d'au moins 10% pour une mousse d'une épaisseur de 1,5 cm, et préférentiellement d'au moins 15%. Ladite mousse peut comporter une couche de passivation apte à la protéger contre le milieu réactionnel du réacteur de photocatalyse et contre sa dégradation ou oxydation résultant directement de la résultant de la présence du photocatalyseur. Ladite mousse peut comporter des nanotubes ou nanofibres, qui constituent, ou qui supportent en tant que phase intermédiaire, la phase photocatalytiquement active. Lesdits nanotubes ou nanofibres sont préférentiellement sélectionné parmi le TiO₂ et les titanates. Le diamètre extérieur de ces nanotubes ou nanofibres peut être compris entre 10 nm et 1000 nm, préférentiellement entre 10 nm et 160 nm, et encore plus préférentiellement entre 10 nm et 80 nm.

La phase photocatalytiquement active doit être un semi-conducteur, et peut être un chalcogénure (tel qu'un oxyde, sulfure, séléniure). Plus particulièrement, elle peut être sélectionnée dans le groupe constitué par : les oxydes métalliques tels que WO₃, ZnO, TiO₂ et SnO₂ ; les titanates, les sulfures ou séléniures métalliques, éventuellement dopés, tels que CdS, CdSe, ZnS, ZnSe et WS₂ ; les semi-conducteurs de type III-V, éventuellement dopés, tels que GaAs et GaP. Le semi-conducteur peut être dopé, modifié en surface ou dans le volume, ou couplé avec d'autres matériaux qui sont avantageusement des semi-conducteurs.

Un procédé pour la fabrication d'un photocatalyseur à base de mousse alvéolaire en carbone comprend les étapes suivantes :
(a) on approvisionne une préforme en mousse alvéolaire polymère carbonisable;
(b) on imprègne ladite préforme d'une résine polymère carbonisable ;
(c) on polymérise ladite résine polymère ;
(d) on transforme ladite préforme et ladite résine polymérisée en carbone ;
(e) on dépose une phase photocatalytiquement active, de préférence sélectionnée parmi les semi-conducteurs dans le groupe constitué par :
   - les oxydes métalliques tels que WO₃, ZnO, TiO₂ et SnO₂,
   - les titanates,
   - les sulfures ou séléniures métalliques, tels que CdS, CdSe, ZnS, ZnSe et WS₂,
   - les semi-conducteurs de type III-V, tels que GaAs et GaP,
ces phases photocatalytiquement actives étant éventuellement dopées ou greffées d'éléments de transfert de charge (tels que de chromophores et/ou de nanoparticules (« quantum dots »), et/ou d'un second matériau semi-conducteur absorbant dans le spectre visible ou ultra-violet (UV) et capable de transférer la charge sur le premier semi-conducteur ou inversement.

Entre les étapes (d) et (e), on peut déposer des nanotubes ou nanofibres, de préférence en TiO₂ ou titanate, le dépôt desdits nanofibres ou nanotubes en TiO₂ pouvant éventuellement remplacer le dépôt du photocatalyseur en étape (e).
La phase photocatalytiquement active peut être déposée par exemple par l'une des techniques suivantes :
- à partir d'une suspension de particules cristallisées, appliquée de préférence par trempage et imprégnation, par voie aérosol ou par goutelettes,
- par synthèse sol-gel,
- par dépôt à partir d'une phase vapeur,
- par le dépôt de couches successives de polyélectrolytes,
- par la technique Langmuir-Blodgett.
Le photoréacteur selon l'invention comporte au moins un photocatalyseur tel que décrit ci-dessus. Ce photocatalyseur comprend un élément d'enveloppe étanche aux liquides et aux gaz, au moins une pièce d'un photocatalyseur tel que décrit ci-dessus à l'intérieur dudit élément d'enveloppe, et au moins une source d'un rayonnement lumineux. Ladite au moins une pièce de photocatalyseur a une forme annulaire.
Ledit photoréacteur comprend une pluralité de N pièces annulaires d'un photocatalyseur, et ledit rayonnement lumineux est introduit dans le diamètre intérieur desdites pièces annulaires, et lesdites pièces annulaires présentent un diamètre intérieur alternativement différent, de manière à ce que toutes les pièces de numéro d'ordre pair présentent le même diamètre intérieur d₁, et toutes les pièces de numéro d'ordre impair présentent le même diamètre intérieur d₂. Lesdites pièces annulaires peuvent être séparées d'un espace vide ou d'une pièce optiquement transparente à au moins une partie dudit rayonnement lumineux utilisé.

Un autre objet de l'invention est l'utilisation d'un tel photoréacteur selon l'invention pour catalyser des réactions chimiques en phase liquide.
Un dernier objet de l'invention est l'utilisation d'un tel photoréacteur selon l'invention pour l'inactivation ou dégradation d'agents biologiques. »

### Description

D'une manière générale, dans le présent document, le terme « surface spécifique développée » fait référence au rapport entre la surface développée (m²) et le volume occupé (m³) : ce paramètre définit la surface exposée au flux par unité de volume. Le volume occupé est défini par les cotes externes de la pièce, comme si elle était pleine.
La « porosité » d'un matériau est habituellement définie par référence à trois catégories de pores qui se distinguent par leur taille : la microporosité (diamètre inférieur à environ 2 nm), la mésoporosité (diamètre compris entre environ 2 et environ 50 nm) et la macroporosité (diamètre supérieur à environ 50 nm).

Le terme « mousse alvéolaire » désigne une mousse à porosité ouverte qui présente à la fois une très faible densité et un grand volume poreux. La taille de l'ouverture de pores est variable. Une telle mousse présente une très faible microporosité. La mésoporosité est liée essentiellement aux ponts qui forment les alvéoles. La macroporosité ouverte d'une telle mousse peut varier de 30 à 95%, notamment de 50 à 90%, et sa densité volumique peut être comprise entre 0,05 g/cm³ et 0,5 g/cm³. D'une manière générale, pour son application comme support de catalyseur ou catalyseur, en dessous d'une densité de 0,05 g/cm³, on rencontre des problèmes de tenue mécanique de la mousse, alors qu'au-dessus de 0,5 g/cm³, le volume poreux alvéolaire va être réduit et la perte de charge va augmenter sans procurer un avantage fonctionnel. Avantageusement, la densité est comprise entre 0,1 et 0,4 g/cm³.
Selon l'acception générale du terme mousse (« foam » en anglais), celle-ci n'est pas forcément alvéolaire. Dans ce sens plus général du terme « mousse », elle peut aussi simplement comporter des bulles (à l'instar des mousses métalliques ou des mousses de ciment obtenues par l'ajout de poudres d'aluminium qui, en réagissant avec le ciment liquide, forment des bulles de gaz). Une telle mousse n'est pas alvéolaire. D'une manière générale, les mousses alvéolaires poreuses sont décrites par quatre grandeurs caractéristiques principales : la taille des fenêtres (Phi), la taille des alvéoles (a), la taille des ponts (ds) et la porosité (epsilon) ; la porosité (epsilon) est égal à 1 -Vₛ / Vₘₒᵤₛₛₑ, où Vₘₒᵤₛₛₑ représente le volume occupé macroscopiquement par la mousse (ce volume étant défini par les cotes de la pièce de mousse, comme s'il s'agissait d'une pièce pleine), et Vₛ représente le volume de matière constituant la pièce de mousse.
Ces quatre paramètres sont souvent liés deux par deux : par exemple : phi/a = f(epsilon), ou ds/a=f(epsilon).

D'une manière générale, dans le présent document, on entend par « matériau carboné », on entend ici tout matériau organique, naturel ou synthétique, tels que : les polymères à base de chaînes de carbone, pouvant comporter des hétéroatomes dans la chaîne ou en tant que substituants ; les matériaux obtenus par dégradation (thermique par exemple) partielle de polymères à base de chaînes de carbone. En ce sens, les carbures et le carbone pur (obtenu par exemple par pyrolyse totale de matériaux carbonés) ne font pas partie des « matériaux carbonés ».

D'une manière générale, dans le présent document, on entend par « agent biologique» des entités de nature biologique, généralement de faible taille, typiquement comprise entre 0,01 µm et 10 µm, et susceptibles de pouvoir être véhiculées par un courant gazeux ou liquide. Ainsi, les agents biologiques à inactiver selon le procédé selon l'invention peuvent notamment être des bactéries (telles que les bactéries du genre *Legionella,* par exemple *Legionella pneumophila*), des virus, des spores de champignons, des spores bactériennes ou un mélange de ces entités.
Par « agent biologique inactivé », on entend ici un agent biologique ayant perdu une activité biologique, et notamment ayant perdu sa capacité de réplication ou de reproduction, ou, dans le cas des virus, sa capacité d'infection ou de contamination. Ainsi, une bactérie inactivée n'est plus capable de développer une colonie après mise en culture dans un milieu adapté, et un virus inactivé n'est plus capable de se reproduire dans une cellule adaptée.

Le problème peut être résolu en utilisant des mousses alvéolaires en carbone, qui présentent une transmittance suffisante à la lumière, une perte de charge très faible et une porosité élevée. Selon l'invention, les mousses doivent présenter une taille moyenne d'alvéoles comprise entre 2500 µm et 5000 µm ; de manière surprenante, malgré la taille élevée des alvéoles, de telles mousses présentent une activité catalytique suffisante. Au-dessous de 2500 µm, la transmission optique des mousses devient trop faible pour des applications en couche épaisse (telles que requises dans la plupart des réacteurs industriels). Au-dessus de 5000 µm, l'efficacité de conversion diminue considérablement.

Le fait que des mousses avec une taille d'alvéoles si grande donnent des bons résultats est surprenant, à la fois par rapport aux monolithes en céramique poreuse, et par rapport aux mousses alvéolaires présentant des alvéoles de petite taille. En effet, compte tenu des facteurs hydrodynamiques, l'utilisation de mousses alvéolaires à grande taille d'alvéoles en catalyse ou en filtration, et surtout en photocatalyse, ne paraît *a priori* pas intéressante.

En effet, selon les constatations des inventeurs, les mousses alvéolaires présentent un intérêt en termes de performances si leurs quatre facteurs principaux sont modulés de manière adéquate. En particulier :
- La surface spécifique développée par les mousses alvéolaires est un facteur important. Dans le cadre de la présente invention, on préfère une surface développée d'au moins 100 m⁻¹ et de préférence comprise entre 100 m⁻¹ et 3000 m⁻¹.
- Le pouvoir de filtration ou d'impactage des mousses alvéolaires est surprenamment important. On parle de pouvoir de filtration pour des particules (donc des microorganismes: virus, bactéries, spores, etc...) et d'impactage pour des molécules chimiques. On peut en général définir quatre forces agissant sur ce pouvoir de filtration ou d'impactage : le mouvement brownien, les forces de gravité, les forces d'intersection, les forces d'impaction directe. Les inventeurs ont trouvé que ces forces ont un impact plus marqué pour des tailles de ponts (ds) plus petits. Les forces prépondérantes dépendent des cas : s'il s'agit de molécules chimiques ou de microorganismes, et même si il s'agit de virus (par exemple 40 nm de taille) ou des bactéries (par exemple 1 µm x 3 µm de taille).
- Le transfert de matière gaz-solide ou liquide-solide est élevé. Les inventeurs ont constatés que de manière surprenante, ce transfert de matière s'améliore pour des tailles d'alvéoles plus élevées.
- La transmission de la lumière dans les mousses doit être élevée pour des applications de photocatalyse.

Sachant que les différentes grandeurs caractéristiques sont liées entre-elles, le gain en performances lors de l'utilisation des mousses alvéolaires en photocatalyse résulte d'une optimisation entre ces différents paramètres. Cet optimum est représenté principalement par des mousses alvéolaires dont la taille moyenne des alvéoles est comprise entre 2500 µm et 5000 µm, et plus particulièrement entre 3000 µm et 5000 µm.

Un autre avantage des mousses alvéolaires de carbone par rapport aux monolithes céramiques poreux est également que chacun des quatre paramètres (i.e. la taille des fenêtres (Phi), la taille des alvéoles (a), la taille des ponts (ds) et la porosité (epsilon)) est contrôlable (modulable), ce qui n'est pas le cas des monolithes.

Les mousses utilisées dans le cadre de l'invention doivent présenter une transmission optique dans le spectre visible et proche UV suffisante pour leur utilisation en tant que photocatalyseur ou support de photocatalyseur. On préfère que la transmission optique globale soit d'au moins 10% pour une épaisseur de 1,5 cm de mousse alvéolaire et pour une lumière d'une longueur d'onde comprise entre 400 et 750 nm.

D'une manière générale, pour son application comme support de catalyseur ou catalyseur dans le cadre de la présente invention, en dessous d'une densité de 0,05 g/cm³, on rencontre des problèmes de tenue mécanique de la mousse, alors qu'au-dessus de 0,5 g/cm³, le volume poreux alvéolaire va être réduit et la perte de charge va augmenter sans procurer un avantage fonctionnel. Avantageusement, la densité de la mousse alvéolaire utilisée dans le cadre de la présente invention est comprise entre 0,1 et 0,4 g/cm³.

L'invention peut être réalisée avec différents types de mousses alvéolaires.

### a) Mousses alvéolaires de carbone

Ces mousses peuvent être préparées par l'imprégnation d'une préforme alvéolaire en mousse polymère, préférentiellement une mousse de polyuréthane (PU) (de telles mousses existent dans le commerce) par un mélange de résine formo-phénolique, suivi d'un séchage (typiquement à la température ambiante pendant une nuit) puis de la polymérisation de la résine par étuvage (typiquement à 150°C pendant environ 2 heures). Ensuite, on effectue une pyrolyse (typiquement à environ 700°C pendant environ 2 heures sous flux de gaz inerte, avantageusement de l'argon) pour transformer la mousse de polymère réticulée en mousse de carbone.

### b) Dépôt de nanofibres ou nanotubes

Ce dépôt est optionnel. Les nanotubes ou nanofibres peuvent être déposées sur la mousse alvéolaire de carbone, selon des techniques connues de l'homme du métier. Par exemple, on peut les déposer à l'aide de techniques décrites ci-dessous sous lettre c) (« première méthode »), qui convient également pour tous types de nanotubes et nanofibres.

Le dépôt de nanotubes de titanates peut être fait de la même manière que le dépôt de nanotubes de TiO₂, décrit ci-dessous sous lettre c) (« première méthode »), qui convient également pour tous types de nanotubes et nanofibres. Les nanotubes de titanates sont préparés par traitement hydrothermique (avantageusement à une température comprise entre 110 et 145°C, typiquement à 130°C) d'une poudre de TiO₂ dans une base forte (typiquement NaOH) concentrée (typiquement 10 M) dans un autoclave.

Ensuite, on lave, on sèche et on calcine à une température comprise entre 350°C et 450°C (typiquement 380°C). Selon un mode de réalisation, on ajoute 1 g de TiO₂ pulvérulent à 50 mL d'une solution de NaOH (10 M) dans un autoclave en téflon ®. L'ensemble est agité pendant une heure puis laissé à 130°C pendant 48 heures. La poudre blanche obtenue est ensuite filtrée sous vide et lavée avec HCl (2 M) jusqu'à l'obtention de la neutralité, rincée à l'eau distillée, puis séchée toute la nuit à 110°C, et on calcine à 380°C.

### c) Dépôt du photocatalyseur

Le photocatalyseur peut être déposé directement sur la mousse alvéolaire, passivée ou non, ou sur une phase intermédiaire, notamment unidimensionnelle. Une telle structure intermédiaire unidimensionnelle peut être une structure à l'échelle nanométrique, dans le sens où au moins une dimension de l'objet est limitée à une taille nanométrique (notamment sous les appellations usuelles de nanofibres, nanotubes ou nanowires, sans exclusive, indépendamment de leur nature chimique, même si de tels supports à base de carbone ou d'oxyde sont les plus répandus).
Elle peut également être une structure à l'échelle micronique, dans le sens où au moins une dimension de l'objet est limitée à une taille micronique (notamment sous les appellations usuelles de microfibres ou fibres, sans exclusive, indépendamment de leur nature chimique ; les supports fibreux à base de silice ou quartz étant mentionné ici comme des exemples).

Avantageusement, si une phase intermédiaire est utilisée, la phase photocatalytiquement active est déposée sur les nanotubes ou nanofibres déposés comme décrit ci-dessus.

La phase photocatalytiquement active doit comporter au moins un matériau semi-conducteur dans sa composition chimique.
Par matériau semi-conducteur, on entend au sens de la présente invention, un matériau où les états électroniques ont un spectre de bande comprenant une bande de valence et une bande de conduction séparées par une bande interdite, et où l'énergie nécessaire pour faire passer un électron de ladite bande de valence à ladite bande de conduction est de préférence comprise entre 1,5 eV et 4 eV. A titre de tels matériaux semi-conducteurs, on peut notamment citer les chalcogénures, et plus particulièrement l'oxyde de titane, ou bien encore d'autres oxydes métalliques tels que WO₃, ZnO ou SnO₂, ou bien des sulfures métalliques tels que CdS, ZnS ou WS₂, ou des séléniures tel que CdSe, ou encore d'autres composés tels que GaAs, GaP. Selon la présente invention, on utilise préférentiellement l'oxyde de titane TiO₂ qui conduit à des résultats particulièrement satisfaisants, et qui est peu onéreux.
Au sens de la présente description, le terme « matériau semi-conducteur photoactivé » désigne un matériau semi-conducteur du type précité qui a été soumis à un rayonnement comprenant des photons d'énergie supérieure ou égale à l'énergie nécessaire pour promouvoir les électrons de la bande de valence vers la bande de conduction (énergie dite gap entre les bandes de valence et de conduction).
Ainsi, au sens de la présente description, on entend en particulier par « oxyde de titane photoactivé » un oxyde de titane soumis à un rayonnement comprenant des photons d'énergie supérieure ou égale à l'énergie nécessaire pour promouvoir les électrons de la bande de valence vers la bande de conduction, typiquement un rayonnement contenant des photons d'énergie supérieure à 3 eV, de préférence à 3,2 eV, et en particulier un rayonnement comprenant des longueurs d'ondes inférieures ou égales à 400 nm, par exemple inférieures ou égales à 380 nm. On peut aussi utiliser la lumière visible, si elle permet d'activer le matériau semi-conducteur. Cela est le cas du TiO₂ sous la forme rutile, à titre d'exemple. En cas de besoin, par exemple pour le TiO₂ anatase, on peut greffer des éléments de transfert de charge sur le semi-conducteur ; il peut s'agir de chromophores et/ou de nanoparticules (« quantum dots »), d'un second matériau semi-conducteur absorbant dans le spectre visible et pouvant transférer la charge sur le premier semi-conducteur. A titre d'exemple, on peut utiliser des nanoparticules (d'une dimension typique comprise entre 2 et 10 nm) de CdS. Une autre possibilité pour utiliser du TiO₂ de forme anatase est de le modifier par dopage ; l'anatase conduit à un rendement quantique meilleur que la forme rutile.

A titre de rayonnements, on peut notamment citer les rayonnements fournis par les lampes à rayonnements ultraviolets du type des lampes dites à lumière noire, ou ceux fournis par des Diodes ElectroLuminescentes (DEL en français, LED en anglais).
Il est connu que, dans un matériau semi-conducteur photoactivé, et en particulier dans un oxyde de titane photoactivé, il se crée, sous l'effet d'un rayonnement de type précité, des paires électrons/trous (un trou étant un déficit électronique dans la couche de valence laissé lors du saut d'un électron vers la bande de conduction), ce qui confère au matériau semi-conducteur photoactivé des propriétés d'oxydo-réduction prononcées. Ces propriétés d'oxydo-réduction sont particulièrement prononcées dans le cas de l'oxyde de titane photoactivé, qui sont mises à profit dans de nombreuses applications photocatalytiques de l'oxyde de titane.

Le dépôt de particules photocatalytiques sur la mousse alvéolaire peut être un dépôt discontinu de particules photocatalytiques isolées, ou bien peut consister en un revêtement plus ou moins uniforme recouvrant une partie significative, voire la majorité ou même la totalité de la surface. Le dépôt de la phase photocatalytiquement active peut être effectué directement sur la mousse alvéolaire, ou sur un revêtement intermédiaire déposé sur cette mousse, par exemple des nanofibres ou nanotubes. Les particules photocatalytiques peuvent être composées d'un semi-conducteur unique, ou bien consister en un mélange de phases dont l'une au moins est photocatalytique. Avantageusement, les particules photocatalytiques sont du TiO₂ (dioxyde de titane) ; ces particules peuvent être dopées.

Le dépôt de la phase photocatalytiquement active peut être réalisé par tout procédé approprié. Dans ce qui suit, nous décrivons plusieurs techniques de dépôt, en prenant comme exemple une phase active préférée, le TiO₂. Il est entendu que ces techniques et procédés peuvent être adaptés à d'autres phases photocatalytiquement actives, et notamment à d'autres oxydes et d'autres chalcogénures.

Selon une première méthode, on dépose des particules cristallisées, que l'on met en suspension dans un solvant approprié, puis on les répand sur le substrat constitué par la mousse, par exemple par trempage du substrat. Plus précisément, le dépôt peut être obtenu par imprégnation de la mousse d'une solution contenant des particules de la phase photocatalytiquement active sous forme cristallisée, par exemple un chalcogénure (tel que le TiO₂). Cette imprégnation est suivie d'un séchage pour éliminer le solvant utilisé lors de l'imprégnation.

Selon une deuxième méthode, la synthèse de TiO₂ peut être réalisée directement sur la mousse, en l'imprégnant par une solution contenant le précurseur de TiO₂, selon un mode de synthèse appelée synthèse sol-gel. Ce procédé peut être réalisé selon différentes manières. Dans un mode de réalisation avantageux, on dépose d'abord, à partir d'une solution colloïdale d'un gel amorphe, des particules essentiellement amorphes sur le substrat constitué par la mousse, puis on sèche et on chauffe à une température et pour une durées suffisantes pour transformer ces particules amorphes en cristaux. Toute méthode de type sol-gel dont la mise en oeuvre permet d'obtenir des particules cristallisées peut convenir. La cristallisation sera meilleure à haute température, mais la température ne doit pas dépasser 120 à 140°C pour la mousse de PU et 400 à 450°C pour la mousse de carbone.
Dans un mode de réalisation avantageux de cette méthode, le précurseur de TiO₂ est un alcoxyde de titane, et préférentiellement de l'isopropoxyde de titane. Il s'en suivra alors une étape de séchage puis de calcination, à une température ne dépasant pas les valeurs indiquées ci-dessus, pour cristalliser le matériau sous sa forme TiO₂. Cette technique de sol-gel est applicable à d'autres oxydes métalliques.

Selon une troisième méthode, la synthèse de TiO₂ peut également être réalisée directement sur mousse à partir d'une phase vapeur contenant un précurseur gazeux de TiO₂, en faisant passer un flux gazeux contenant ledit précurseur de TiO₂. Ce précurseur peut être par exemple un alcoxyde de titane ou un chlorure de titane. Ce procédé peut être assisté par plasma, ou peut se dérouler sans plasma. Il s'en suivra alors une étape de séchage puis de calcination pour cristalliser le matériau sous sa forme TiO₂. Cette technique est applicable à d'autres semi-conducteurs photocatalytiquement actifs.

Selon une quatrième technique (appelée «LBL », ce qui signifie Layer-By-Layer), on dépose des couches successives de polyélectrolytes. Cette technique est décrite de manière conceptuelle dans l'article « Fuzzy Nanoassemblies : Toward Layered Polymeric Multicomposites » par Gero Decher, paru dans la revue Science, vol. 277, p. 1232-1237 (1997)). Avantageusement, on dépose au moins huit couches.

Selon une cinquième technique, on dépose des particules de phase photocatalytiquement active (tel que le TiO₂) par la méthode de Langmuir-Blodgett, décrite en tant que telle dans l'article « Préparation and Organized Assembly of Nanoparticuloate TiO2 - Stearate Alternating Langmuir-Blodgett Films » par Lin Song Li et al, paru dans Journal of Colloid and Interface Science, vol 192, p. 275-280 (1997), dans l'article « Préparation of a TiO2 Nanoparticulate Film Using a Two-Dimensional Sol-Gel Process » by I. Moriguchi et al, paru dans la revue Chem. Mater., (1997), p. 1050-1057, et dans l'article « Characterization of TiO2 Nanoparticles in Langmuir-Blodgett Films » par P.J.G. Coutinho, paru dans Journal of Fluorescence (2006), p. 387-392.

La nature exacte de la phase active utilisée selon l'invention pour développer des propriétés photocatalytiques, dans la mesure où elle comporte au moins un matériau activé par le rayonnement lumineux, n'est, en règle générale, pas déterminante pour mettre en oeuvre une réaction ou un procédé photocatalytique.
Ainsi, dans le cas de l'oxyde de titane, par exemple, tout oxyde de titane développant des propriétés photocatalytiques et pouvant être ancré sous forme de particules ou de revêtement sur la mousse peut être utilisé efficacement dans le procédé de l'invention, ce qui constitue encore un avantage du procédé.
Néanmoins, selon un mode de réalisation, l'oxyde de titane utilisé selon le procédé de l'invention contient du TiO₂ de forme anatase, de préférence à raison d'au moins 50%. Ainsi, selon ce mode de réalisation, l'oxyde de titane utilisé peut par exemple être constitué pour l'essentiel (à savoir en général pour au moins 99% en masse, et de préférence pour au moins 99,5% en masse, voire pour au moins 99,9% en masse) de TiO₂ de forme anatase.
L'utilisation de TiO₂ sous forme rutile se révèle également intéressante, dans la mesure où le TiO₂ sous cette forme est photoactivé par le spectre de la lumière visible.
Selon un autre mode de réalisation avantageux, l'oxyde de titane utilisé comprend un mélange de TiO₂ de forme anatase et de TiO₂ de forme rutile, avec une proportion massique d'anatase/rutile de préférence entre 50/50 et 99/1, par exemple entre 70/30 et 90/10, et typiquement de l'ordre de 80/20.
Par ailleurs, notamment pour optimiser les échanges entre le matériau semi-conducteur de type oxyde de titane et le flux réactionnel, il est le plus souvent avantageux que le matériau semi-conducteur utilisé ait une surface spécifique comprise entre 2 et 500 m²/g, de préférence supérieure ou égale à 20m²/g, et encore plus avantageusement au moins égale à 50 m²/g et ce, tout particulièrement lorsqu'il s'agit d'oxyde de titane.
Le matériau semi-conducteur photoactivé qui est utilisé selon l'invention peut se présenter sous différentes formes physiques, en fonction du milieu traité, et notamment en fonction du volume de ce milieu et de la vitesse à laquelle on souhaite mettre en oeuvre le procédé. De façon générale, le matériau semi-conducteur de type oxyde de titane peut être utilisé sous toute forme adaptée à son irradiation par un rayonnement de longueur d'onde permettant sa photoactivation et permettant la mise en contact de l'oxyde de titane à l'état photoactivé avec les molécules du flux réactionnel, sous réserve qu'il soit accessible.

### d) Utilisation en photocatalyse

Plusieurs types de réacteurs photocatalytiques peuvent être utilisés avantageusement. On peut introduire une ou plusieurs pièces de mousse, par exemple de forme cylindrique, dans un élément d'enveloppe formant une paroi étanche aux liquides et aux gaz, ledit élément d'enveloppe étant transparent ou non, à travers lequel est conduit le milieu réactionnel. Ledit élément d'enveloppe peut être un élément tubulaire. Dans le cas d'un élément d'enveloppe transparent, la lumière peut provenir de l'extérieur (i.e. par une lampe extérieure), alors que dans le cas d'un élément d'enveloppe opaque, la lumière doit provenir de l'intérieur (par exemple par une lampe interne, ou par des diodes LED, ou par des dispositifs de type quantum dot), ou doit être apportée à l'intérieur par des fibres optiques. Plusieurs de tels éléments d'enveloppe, par exemple des éléments tubulaires, peuvent être mis en parallèle, éventuellement utilisant une source lumineuse commune (notamment dans le cas de tubes transparents). On peut également utiliser un réacteur à voies multiples, chaque voie étant constituée d'au moins un élément tubulaire, et le réacteur étant pourvu de d'électrovannes permettant de basculer le flux de milieu réactionnel sur au moins un élément(s) tubulaire(s), l'autre (ou les autres) pouvant être régénéré(s) ou échangé(s) pendant que le ou les autre(s) fonctionne(nt).
Dans un autre mode de réalisation, on utilise un réacteur, typiquement tubulaire, de diamètre plus important, par exemple compris entre 10 et 100 cm, dans lequel on introduit plusieurs pièces cylindriques de mousse, ainsi qu'une pluralité de sources lumineuses ; ces dernières sont introduites dans ces pièces de mousse, typiquement sous la forme de lampes de forme allongée (typiquement approximativement cylindrique) ou fibres optiques orientées parallèlement au sens long dudit élément tubulaire. Les pièces de mousse sont avantageusement de forme cylindrique ; elles peuvent avoir la forme d'anneaux. Au sein d'un même réacteur, des pièces de mousse successives peuvent comprendre des photocatalyseurs de nature différente.

Selon l'invention, on introduit dans un élément tubulaire des anneaux de mousses présentant de manière alternée des diamètres intérieurs différents. La figure 3(b) montre de manière schématique cette géométrie pour une configuration comportant 13 anneaux cylindriques de mousse, alternativement de 7 cm de diamètre extérieur et de 2 cm d'épaisseur ; la figure 5 montre les cotes. Au centre des anneaux se trouve une source lumineuse longitudinale. La figure (3a) montre à titre de comparaison un réacteur avec des anneaux cylindriques de mousses de diamètre identique. On a calculé le profil de vitesse dans les deux réacteurs pour un flux de gaz ou de liquide ; le résultat est donné sur la figure 4. On constate ainsi que le profil de vitesse des particules est asymétrique dans le cas où le diamètre intérieur des anneaux n'est pas le même (cas de la figure 3(b)). C'est grâce à cette asymétrie qu'une plus grande partie du flux passe dans les zones les plus éclairées : un tel réacteur présente un taux de conversion plus élevé qu'un réacteur dont le diamètre intérieur est constant. Un réacteur rempli de mousses en alternance peut être préféré au même réacteur rempli intégralement de mousse, sur l'ensemble de sa longueur.
Ceci permet d'augmenter globalement la quantité de lumière reçue en chaque point de la mousse, ce qui peut, dans le cas d'un choix judicieux de l'alternance des mousses, compenser la diminution de la quantité de mousses, et donc de la quantité de phase photocatalytiquement active à l'intérieur du réacteur.
Dans un mode de réalisation illustré schématiquement sur la figure 5, l'espace laissé disponible par le retrait d'un certain nombre de mousses du réacteur est réparti sur l'ensemble du réacteur, de manière que les mousses soient équidistantes à l'intérieur du réacteur. L'équidistance n'est cependant pas une nécessité absolue.

Le photocatalyseur utilisé dans le cadre de l'invention convient pour les réactions en phase gazeuse ou liquide, pour les réactions de type oxydation (par exemple l'oxydation des alcools ou l'oxydation du CO en CO₂), réduction, reformage, décomposition (par exemple de composés organiques volatiles (COV) nuisibles), l'hydrogénation et/ou la déshydrogénation des hydrocarbures ou des composés organiques, la photodissociation de l'eau ou le reformage d'alcools tels que le méthanol. Il convient également pour des oxydations partielles de molécules organiques. Il convient également pour l'oxydation de molécules contenant des hétéroatomes tels que le soufre, le phosphore, l'azote. On cite ici parmi les molécules soufrées le diéthylsulfure, le diméthyl sulfure, le H₂S, le SO₂. On cite ici parmi les molécules phosphorées les molécules organophosporées, telles que les di-méthyl-méthylphosphonates. On cite ici parmi les molécules azotées les méthylamines et l'acétonitrile. Il convient également pour les réactions permettant le traitement des oxydes d'azote (NOₓ).

Le photocatalyseur utilisé dans le cadre de l'invention peut aussi être utilisé comme filtre pour filtrer des agents biologiques, tels que bactéries, virus ou tout autre composé assimilés dans une phase liquide ou gazeuse. Cette activité de filtration est avantageusement accompagnée d'une activité photocatalytique. Les mousses alvéolaires utilisées dans le cadre de l'invention ne retiennent que partiellement ces objets de petite taille, mais leur effet filtrant est suffisant pour conduire à une augmentation du temps de résidence telle que la réaction photocatalytique est plus efficace. Dans le cas des agents biologiques, l'activité photo-catalytique conduit à la mort cellulaire : un tel filtre retient au moins partiellement les agents biologiques et relargue des agents biologiques inactivés.
A titre d'exemple, un tel réacteur peut être installé, de manière très simple, à l'entrée de conduits de climatisation ou d'aspiration d'air de bâtiments ou véhicules. Il peut aussi être utilisé pour purifier des effluents gazeux ou liquides.

### a) Utilisation pour la décontamination biologique

Le photocatalyseur utilisé dans le cadre de l'invention peut être utilisé pour l'inactivation ou la dégradation d'agents biologiques. On connaît déjà des filtres de bactéries à base de mousse de PU (voir l'article « Potential of Silver Nanoparticle-Coated Polyurethane Foam As an Antibacterial Water Filter » par P. Jain et T. Pradeep, paru dans la revue « Biotechnology and Bioengineering, vol 90(1), avril 2005, p. 59 - 62). Mais il ne s'agit pas là d'un procédé catalytique, car la mousse est recouverte de nanoparticules d'argent, dont l'effet bactéricide est connu par ailleurs.

Selon l'invention, on utilise un procédé photocatalytique pour détruire les agents biologiques, qui peuvent aussi être des virus, des bactéries, des spores bactériens, des allergènes, des spores de champignons contenus dans des fluides, gazeux ou liquides. L'avantage de ce filtre photocatalytique est sa fable perte de charge, même pour des épaisseurs importantes (de l'ordre d'une dizaine de centimètres à une centaine de centimètres). Cela permet de traiter des fluides avec des débits (ou vitesses linéaires) importants, tous en assurant une activité filtrante et une activité photocatalytique dans le volume. En revanche, la plupart des milieux photocatalytiques connus montrent des limitations sérieuses. A titre d'exemple, les milieux filtrants bi-dimensionnels, tels que les feutres, les papiers, les tissés, ne permettent pas une pénétration profonde de la matière retenue par le filtre dans le milieu filtrant, et ne peuvent être utilisés en présence de milieux agressifs. Ils peuvent également être limités en termes de volume de flux à traiter, notamment lorsqu'ils sont utilisés en mode de lit léchant pour limiter les problèmes de pénétration de la lumière.

Selon l'invention, on utilise un procédé photocatalytique pour détruire les agents biologiques, qui peuvent aussi être des virus, des bactéries, des spores bactériens, des allergènes, des spores de champignons.

### f) Avantages de l'invention

L'utilisation de mousses tridimensionnelles alvéolaires comme support de photocatalyseur permet de pallier un certain nombre de limitations rencontrés pour la plupart des substrats ou médias photocatalytiques existants, à savoir :
(i) une utilisation en flux traversant avec une perte de charge minime à débit (ou vitesse linéaire) élevé,
(ii) une bonne transmission de la lumière, que l'on peut ajuster en jouant sur la taille des alvéoles,
(iii) un contact intime avec le milieu réactionnel (flux de gaz ou liquide) à traiter, en raison d'une turbulence accrue au contact avec la mousse tridimensionnelle,
(iv) l'utilisation d'un milieu tridimensionnel permet une distance de contact accrue avec le milieu réactionnel lorsque l'on travaille en flux traversant (par exemple approximativement perpendiculairement à la section de la mousse), alors que dans un réacteur tubulaire classique en mode lit traversant, et notamment dans les conditions d'un réacteur piston, la distance de contact, i.e. la distance sur laquelle il peut y avoir contact entre le flux et le revêtement catalytique du réacteur, correspond typiquement à la longueur du réacteur ;
(v) le couplage des propriétés photocatalytiques de la mousse avec ses propriétés de filtration, ces dernières étant ajustables en fonction de la taille des alvéoles,
(vi) d'une manière générale, une souplesse d'adaptation, de modulation et de disposition spatiale de ces mousses, pour les adapter aux différents environnements dans lesquels elles seront utilisées.

Le photocatalyseur utilisé dans le cadre de l'invention peut être produit sous la forme d'une cartouche régénérable.

### Exemples de réalisation

Ces exemples sont donnés à titre d'illustration pour permettre à l'homme du métier de réaliser l'invention. Ils représentent des modes de réalisation particuliers de l'invention ou des aspects partiels de ces modes de réalisation et ne limitent pas sa portée. Certaines caractéristiques techniques sont illustrées à l'aide de mousses polyuréthanes même si celles-ci ne font plus partie de l'invention.

### Exemple 1 : Transmission optique de mousses utilisées pour la mise en oeuvre de l'invention.

On a préparé des mousses à base de différents matériaux, avec une taille moyenne d'alvéoles de 4500 µm. On a déterminé la transmission optique de bloc de différentes épaisseurs avec une lumière émise par une diode d'une longueur d'onde de 455 nm. Pour faire ces mesures, on a utilisé un bloc sous forme d'un disque qui tournait autour d'un axe. La valeur mesurée était une valeur moyenne prise sur différentes orientation de la mousse. Les résultats sont résumés dans le tableau 1.

**Tableau 1**

| Mousse de polyuréthane (taille moyenne des alvéoles : 4500 µm) | | | | | | | |
|---|---|---|---|---|---|---|---|
| Epaisseur [cm] | 0 | 0,43 | 0,96 | 1,46 | 1,75 | 2,26 | 3,25 |
| Transmission [%] | 100 | 45 | 20 | 12 | 9 | 5 | 3 |

| Mousse de PU après polymérisation, mais avant pyrolyse (taille moyenne des alvéoles : 4500 µm) | | | | | | | |
|---|---|---|---|---|---|---|---|
| Epaisseur [cm] | 0 | 0,61 | 1,13 | 1,72 | 2,36 | 2,96 | 3,5 |
| Transmission [%] | 100 | 47 | 23 | 9 | 7 | 3 | 2 |

| Mousse de carbone (taille moyenne des alvéoles : 4500 µm) | | | | | | | |
|---|---|---|---|---|---|---|---|
| Epaisseur [cm] | 0 | 0,46 | 0,86 | 1,34 | 1,83 | 2,36 | 2,56 |
| Transmission [%] | 100 | 42 | 22 | 9 | 8 | 4 | 2 |

| Mousse de carbone + TiO₂ (taille des alvéoles : 4500 µm) | | | | | | | |
|---|---|---|---|---|---|---|---|
| Epaisseur [cm] | 0 | 0,46 | 0,86 | 1,34 | 1,83 | 2,36 | 2,56 |
| Transmission [%] | 100 | 38 | 24 | 11 | 8 | 4 | 3 |

Dans un autre essai, on a mesuré la transmittance optique (à 455 nm) de blocs d'une mousse polyuréthane avec une taille d'alvéoles de 4800 µm ou de 1900 µm pour différents épaisseurs. Ces résultats sont indiqués dans le tableau 2.

**Tableau 2**

| Taille d'alvéoles [µm] | Epaisseur [m] | Transmittance [%] |
|---|---|---|
| 1900 | 0,005 | 0,2389 |
| 1900 | 0,01 | 0,0571 |
| 1900 | 0,015 | 0,0136 |
| 4800 | 0,015 | 0,2498 |
| 4800 | 0,02 | 0,1573 |
| 4800 | 0,025 | 0,0991 |
| 4800 | 0,035 | 0,0393 |
| 4800 | 0,04 | 0,0247 |

### Exemple 2: Perte de charge de mousses polyuréthanes utilisées pour la mise en oeuvre de l'invention.

On a mesuré la perte de charge d'un écoulement d'air sec (masse volumétrique : 1,18 kg/m⁻³, viscosité cinématique 1,84x10⁻⁵ Pa s, température 25°C) dans un bloc d'une mousse polyuréthane d'une épaisseur de 8,00 cm avec taille d'alvéoles de 4800 µm, pour différentes vitesses de l'air. Ces résultats sont résumés dans le tableau 3.

**Tableau 3**

| | | | | | | | | | | |
|---|---|---|---|---|---|---|---|---|---|---|
| Vitesse [m/s] | 0,60 | 2,07 | 3,50 | 3,32 | 4,42 | 4,96 | 5,93 | 6,18 | 7,11 | 7,69 |
| Perte de charge [mBar] | 0,10 | 0,70 | 1,70 | 2,20 | 3,90 | 6,00 | 8,80 | 10,00 | 13,00 | 15,00 |
| Perte de charge(*) [mBar/m] | 125 | 875 | 2125 | 2750 | 4875 | 7500 | 11000 | 12500 | 16250 | 18750 |
| (*) Perte de charge par épaisseur de mousse traversée par l'écoulement d'air | | | | | | | | | | |

### Exemple 3 : Préparation d'une mousse de carbone.

Une mousse de carbone a été préparée par imprégnation d'une mousse de polyuréthane (PU) commerciale avec une taille des alvéoles > 4 800 µm par un mélange de résine formo-phénolique. Cette imprégnation était suivie d'un séchage à température ambiante durant une nuit, puis d'un étuvage à 150°C pendant 2h. La pyrolyse a été effectuée à 700°C pendant 2h (montée 2°C/min sous un flux d'argon à 100 mL/min).

Lors de certains de ces essais, on a utilisé des mousses en PU sous la forme de cylindres. On a par exemple découpé des cylindres d'un diamètre externe de 4,2 cm. Après imprégnation par la résine phénolique, le diamètre du cylindre avait augmenté pour atteindre 5,0 cm. On a alors perforé le cylindre pour obtenir une mousse sous la forme d'anneaux (diamètre interne de 3,0 cm). Durant le traitement de pyrolyse, la mousse a subi un retrait, et l'anneau de mousse carbonée possède alors un diamètre externe de 4,0 cm et un diamètre interne de 2,0 cm.

### Exemple 4 : Dépôt de TiO₂.

Sur douze anneaux de mousse de carbone (masse totale : 11.95 g) préparés selon l'exemple 3, on a déposé du TiO₂ de la manière suivante :

On a mis en suspension 0,787 g de TiO₂ pulvérulente dans une solution éthanol / eau (50/50 en volume) et passé cette suspension aux ultrasons pendant 4 h à la température ambiante (40 mL de solution). Après arrêt de l'agitation, on a trempé chaque anneau de mousse dans la solution, à raison de 3-4 bains successifs alternés par un séchage pendant 30 min à température ambiante. A la fin de cette séquence, on a réalisé un dernier séchage à l'étuve pendant 12 h à 100°C.

Dans une variante de ce procédé, on mis en suspension 3 g de TiO₂ dans 60 mL d'acétone et passé la suspension très brièvement aux ultrasons à la température ambiante. Puis on a placé la suspension à -4°C sous légère agitation mécanique. Après l'arrêt de l'agitation, on trempé chaque anneau de mousse dans la solution, à raison de deux bains successifs alternés par un séchage pendant environ 30 minutes à la température de -4°C. Cette variante implique une évaporation plus lente et conduit à un dépôt plus homogène et plus stable.

### Exemple 5: Préparation d'une mousse de PU passivée et dépôt de la phase photocatalytiquement active (hors invention).

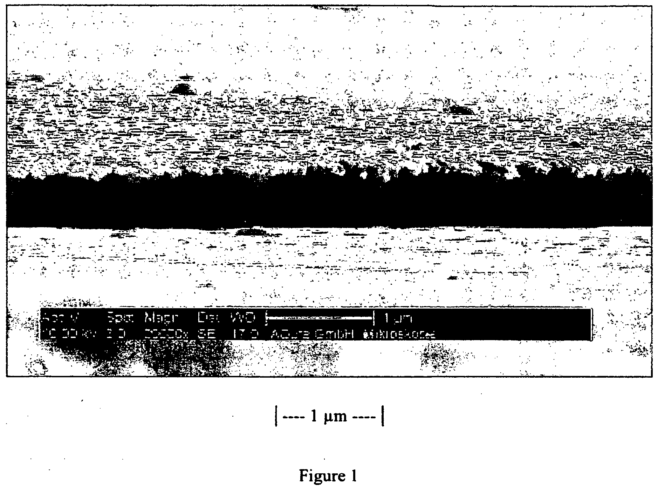

La mousse de PU (du même type que celle décrite dans l'exemple 3) a cette fois été directement passivée, pour la protéger de la dégradation photocatalytique, à l'aide de plusieurs couches formées grâce à un polysiloxane (Sivo 110™, Dynasylan, Evonik). Le Sivo 110™ est un sol-gel aqueux de silice dont une partie des fonctions silanols (Si-OH) ont été modifiées par des fonctions silanes (Si-H), et d'autres par des fonctions époxy réactives, permettant la polymérisation en film dense (voir figure 1).

On a utilisé des anneaux en PU préparés comme décrit dans l'exemple 4. Chaque anneau a été immergé dans une solution à 50% volumique de Sivo 110 : Ethanol. On a ensuite ouvert les alvéoles par un bref passage à l'air comprimé, et puis on a polymérisé pendant 15 min à 120°C sous air. Cette séquence immersion - polymérisation a été réitérée trois fois. Ensuite, on a déposé une dernière couche de ce polymère, qui doit permettre d'ancrer solidement les grains de photocatalyseur au support : après immersion de l'anneau dans une solution à 50% volumique de Sivo 110 : Ethanol, on a laissé évaporer l'éthanol à l'air libre. Il reste un film visqueux sur l'anneau.
Sur cette mousse passivée, on a ensuite déposé un photocatalyseur selon deux modes de réalisation différents :
(a) Voie « poudre » :
   Les anneaux ont été plongés dans un excès de poudre du photocatalyseur à déposer, et sousmis à une polymérisation à 120°C pendant 30 minutes à l'air.
(b) Voie « aqueuse » :
   Les anneaux ont été trempés dans une suspension aqueuse du photocatalyseur (par exemple TiO₂), sous agitation magnétique.

### Exemple 6 : Essais photocatalytiques (Oxydation du méthanol en CO₂ et H₂O).

Dans un réacteur tubulaire (proche de celui décrit dans la publication « Photocatalytic oxydation of butyl acetate on vapor phase on TiO2, Pt/TiO2 and WO3/TiO2 catalysts » par V. Keller et al., parue dans Journal of Catalysis, vol. 215, p. 129-138 en 2003), on a réalisé des essais catalytiques sur différentes mousses, selon deux modes différents, appelés ici « Réacteur structuré » (selon l'invention) et (à titre de comparaison) « Réacteur tubulaire classique ». Toutes les expériences sont réalisées en flux sec.

Pour les deux modes, l'enveloppe extérieure du photoréacteur était un tube en Pyrex de longueur 300 mm et de diamètre 42 mm, au centre duquel était située la source de lumière, à savoir une lampe de type lumière noire (blacklight), fournissant de la lumière UV-A de puissance 8W (fournisseur Philips).

Les tests ont été effectués de la manière suivante :
Le flux d'entrée a été stabilisé en débit et concentration de méthanol sur le by-pass. Puis le même flux a été basculé sur le photoréacteur, dans le noir (lumière UV-A éteinte). Après une période d'adsorption dans le noir (plus ou moins longue selon les conditions expérimentales), pendant laquelle du méthanol s'adsorbe sur le catalyseur, le flux est revenu à sa valeur initiale, et la lampe UV-A a alors été allumé. Les performances photocatalytiques ont alors été suivies par microchromatographie en phase gazeuse.

En raison des flux utilisés et du volume du photoréacteur (volume interne du tube en Pyrex auquel on soustrait le volume occupé par la lampe), la vitesse linéaire du flux gazeux était de 8 cm/s.
On a travaillé sous un flux d'air sec avec un débit de 4,32 L/min, en utilisation une concentration en méthanol de 1200 ppm (volumique). Pour ce faire, un flux d'air (débit 40 mL/min) a été passé dans un saturateur contenant du méthanol liquide (fournisseur Carlo Erba, pureté > 99,9%) à une température de 20°C. Le flux d'air s'est chargé en méthanol, puis a été dilué dans un flux d'air sec porteur (débit 4,28 L/min). Le flux total avait alors un débit de 4,32 L/min.

Les essais comparatifs avec un « réacteur tubulaire (annulaire) classique » ont été effectués de la manière suivante :
La quantité désirée de poudre de TiO₂ a été mise en suspension dans une solution éthanol / eau (50/50 en volume) et a été passée aux ultrasons pendant 4 h à température ambiante (entre 1g et 4g dans 40 mL de solution). La suspension a alors été dispersée sur la surface interne du réacteur tubulaire en une étape, avec un séchage simultané à l'aide d'un sèche-cheveux. Un séchage final a été effectué sous air à l'étuve à 100°C pendant 12 h. Les résultats suivants ont été obtenus avec un photocatalyseur déposé sur mousse alvéolaire de carbone selon l'invention :
Avec le réacteur tubulaire classique (tableau 4) :

**Tableau 4**

| | | | | | |
|---|---|---|---|---|---|
| Masse [g] | 0,5 | 1 | 2 | 3 | 4 |
| Conversion du méthanol [%] | 12 | 16 | 23 | 24 | 25 |

Avec le réacteur structuré (tableau 5) :

**Tableau 5**

| | | | |
|---|---|---|---|
| Masse [g] | 0,8 | 3,5 | 7 |
| Conversion du méthanol [%] | 26 | 40 | 52 |

### Exemple 7 : Essais sur microorganismes

On a utilisé un photoréacteur tubulaire (300 mm de long pour 70 mm de diamètre intérieur) en acier galvanisé qui comportait un revêtement de TiO₂ sur la surface interne de l'enveloppe tubulaire. Ce dernier revêtement a été préparé de la manière suivante :
(i) Sur la surface interne du tube, on a formé un film avec une solution à 50% volumique de SIVO 110 : Ethanol.
(ii) Ensuite on a polymérisé ce film à 180°C pendant 15 minutes.
(iii) On a répété l'étape (i).
(iv) On a laissé évaporer l'éthanol à l'air libre.
(v) On a saupoudre l'intérieur du tube avec un excès de photocatalyseur.
(vi) On a polymérisé à l'air à 180°C pendant 15 minutes.

Dans ce réacteur, on a ensuite effectué des essais d'inactivation biologique de bactéries de type *Legionella pneumophila* avec différents types de mousses. Le débit était de 5 m³/h, en mode « passage simple » (single pass). La lampe était une lampe UV-A 8 Watt de type « Lumière noire » (Blacklight Tube, fourni par Philips). Pour certains essais, on a posé dans le réacteur une mousse PU selon l'invention, passivée par un dépôt de SIVO tel que décrit à l'exemple 6. Les résultats sont résumés sur le tableau 6. Le paramètre LRV (appelé en anglais Logarithmic Réduction in Viability) indique la réduction logarithmique de la viabilité, exprimée comme le logarithme du rapport entre la fraction des microorganismes vivant en entrée et la fraction de microorganismes vivants en sortie du réacteur : LRV = log (%vivant_{Entrée} / %vivant_{Sortie}). La mousse était la même que celle de l'exemple 5.

**Tableau 6**

| Photoréacteur | LRV | % de survie après un passage |
|---|---|---|
| Tubulaire, sans mousse | 0,4 | 79% |
| Tubulaire avec mousse alvéolaire PU avec SIVO comportant du TiO₂ déposé par la voie aqueuse | 2,5 | 0,3% |
| Tubulaire avec mousse alvéolaire PU avec SIVO comportant du TiO₂ déposé par la voie « poudre » | 3,1 | 0,08% |

### Exemple 8 : Dépôt de photocatalyseur « couche par couche ».

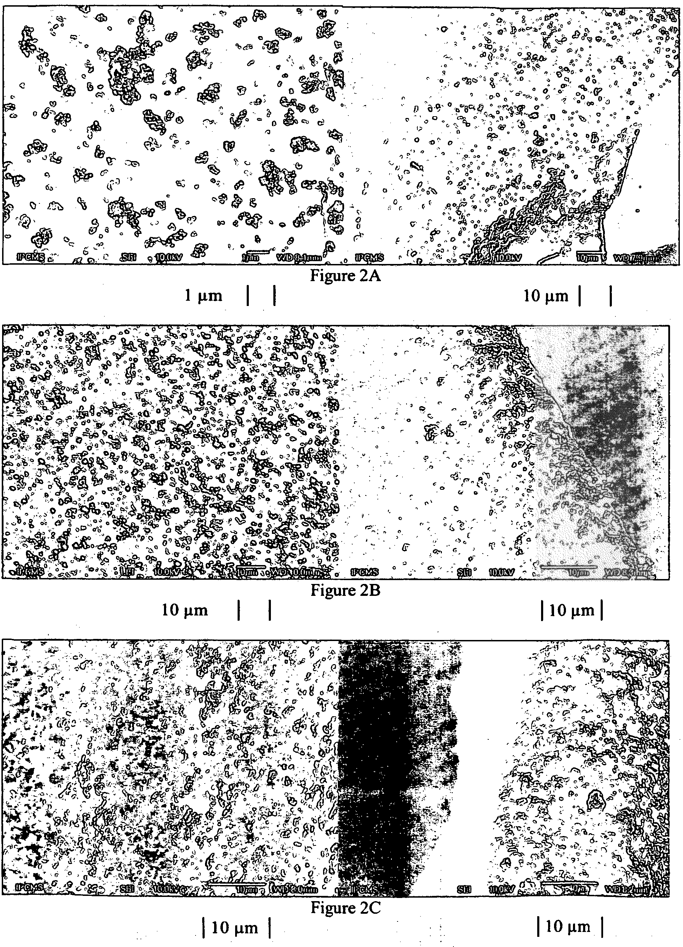

Ces essais ont été effectuées sur des mousse de PU passivées (hors invention) et des mousses de carbone.
Chaque couche a été déposée de la manière suivante :
On a trempé le substrat (mousse) dans une solution de PEI (Polyethylèneimine) pendant 20 min (concentration de PEI de 8.24 g/L). On a ensuite trempé le substrat dans 40 mL d'une solution TiO₂ P25 (eau : éthanol à 50:50 volumiques, à raison de 10 g de P25/L, pH = 8) pendant 20 min. Cette étape était suivie de deux étapes de trempage (lavage) pendant 10 minutes dans l'eau distillée (40 mL). Chaque étape s'est déroulée sous agitation orbitalaire.
Plusieurs couches ont été ainsi déposées. Les figures 2 A, B et C montrent chacune deux micrographies des exemples 9A (5 couches), 9B (10 couches) et 9C (15 couches) ; tous ces dépôts ont été faits sur mousse de carbone (taille moyenne des alvéoles : 4800 µm, masse de la pièce environ 1 g).
Ces mousses montraient des performances catalytiques similaires à celles essayées dans l'exemple 7.

### Exemple 9 : Dépôt de photocatalyseur par phase vapeur.

On a déposé à partir d'une phase gazeuse (méthode CVD) un précurseur de titane sur des mousses de type différent. D'abord, on a fait passer un flux d'air chargé d'éthanol pendant 1 h à un vide de 60 mbar. Puis, on a fait passer un flux d'air chargé en vapeur de TTIP pendant 3h à un vide de 60 mbar. Et enfin, on a de nouveau fait passer un flux d'air chargé en vapeur d'eau pendant 4h à un vide de 60 mbar.

### Exemple 10 : Essais photocatalytiques (Oxydation du méthanol en CO₂ et H₂O)

Cet exemple est un complément à l'exemple 6. Les résultats ont été obtenus avec un photocatalyseur déposé sur mousse alvéolaire de carbone (« réacteur structuré ») ou avec un réacteur tubulaire connu sans mousse :
Avec le réacteur structuré selon l'invention (tableau 7) :

**Tableau 7**

| Débit d'air sec [L/min] | Vitesse [cm/s] | Temps de séjour [s] | Concentration volumique de méthanol | Masse [g] | 0,8 | 3,5 | 7 |
|---|---|---|---|---|---|---|---|
| 4,32 | 8,2 | 3,18 | 1200 ppm | Conversion du méthanol [%] | 26 | 40 | 52 |

Avec le réacteur tubulaire classique (tableau 8) :

**Tableau 8**

| | | | | | | | | | | | | | |
|---|---|---|---|---|---|---|---|---|---|---|---|---|---|
| Débit d'air sec [L/min] | Concentration volumique de méthanol | Vitesse [cm/s] | Temps de séjour [s] | Masse [9] | 0 | 0,21 | 0,38 | 0,76 | 1,53 | 2 | 2,3 | 3 | 4 |
| 4,32 | 1200 ppm | 8,2 | 3,18 | Conver sion du méthanol [%] | 0 | 7,5 | 14,6 | 24 | 23 | 23 | 23 | 24 | 25 |

Avec le réacteur tubulaire Quartzel ® : conversion de 27%
Avec le réacteur tubulaire « Ahlstrom » : conversion de 19%

Dans le cas du mode de test « Réacteur tubulaire Ahlstrom ® », un papier photocatalytique Ahlstrom ® (référence 1048) commercialisé par la société Ahlstrom a été placé de manière circulaire sur la paroi interne du tube. La dimension du papier était : L x I = 260 mm x 126 mm.
Dans le cas du mode de test « Réacteur tubulaire Quartzel® », un feutre photocatalytique Quartzel® commercialisé par la société Saint-Gobain a été placé de manière à entourer la lampe centrale et à remplir l'espace disponible dans le réacteur. La dimension du feutre était : L x I = 260 mm x 126 mm, soit une surface totale de Quartzel® de 32 760 mm².

### Exemple 11 : Essais photocatalytiques (Oxydation du méthanol en CO₂ et H₂O).

Cet exemple est une variante de l'exemple 6. On a travaillé sous un flux d'air sec avec un débit allant de 4,32 L/min à 21,7 L/min en utilisation une concentration en méthanol allant de 10 ppm à 420 ppm (volumique). Pour ce faire, un flux d'air sec adéquat a été passé dans un saturateur contenant du méthanol liquide (fournisseur Carlo Erba, pureté > 99,9%) à une température de 20°C. Le flux d'air a été chargé en méthanol, puis dilué dans un flux d'air sec porteur de manière a obtenir le flux total recherché à la teneur en méthanol recherchée.

Les résultats suivants (tableau 9) ont été obtenus en mettant en parallèle deux réacteurs tels que décrits ci-dessus (les valeurs en pourcent expriment le rendement de conversion du méthanol) :

**Tableau 9**

| Concentration volumique en méthanol [ppm] | | 420 | 210 | 45 | 10 |
|---|---|---|---|---|---|
| Débit 4.3L/min | Mousse PU | 96% | 97% | | |
| Vitesse/réacteur = 4 cm/s | Quartzel® | 86% | 85% | | |
| Temps de séjour = 6,4 s | | | | | |
| | Tubulaire classique | 40% | 52% | | |
| Débit 11,4L/min | Mousse PU | | 88% | 100% | |
| Vitesse/réacteur = 11 cm/s | Quartzel® | | 67% | 72% | |
| Temps de séjour = 2,4 s | | | | | |
| | Tubulaire classique | | 18% | 21% | |
| Débit 21.7 L/min | Mousse PU | | 57% | 88% | 100% |
| Vitesse/réacteur = 20,5 cm/s | Quartzel® | | 47% | 47% | 40% |
| Temps de séjour = 1,3 s | | | | | |
| | Tubulaire classique | | <1% | 1% | 2% |

Les dimensions du feutre photocatalytique Quartzel® utilisé pour ces tests correspondaient à une teneur en TiO₂ de 5,1g à l'intérieur des deux réacteurs.
Dans le cas des mousses alvéolaires de PU selon l'invention chargées en TiO₂, la teneur en TiO₂ n'était que de 2,6 g sur l'ensemble des deux réacteurs.

Cet exemple montre notamment que le bénéfice d'utiliser les mousses s'accroît à la fois par rapport au feutre photocatalytique Quartzel® et par rapport au réacteur tubulaire classique, lorsque le débit augmente (ce qui correspond aussi à une augmentation de la vitesse en m/s)

### Exemple 12 : Etude de l'influence de la taille des alvéoles sur la transmission optique des mousses utilisées pour la mise en oeuvre de l'invention.

Cet exemple est similaire à l'exemple 2, mais on a utilisé ici une lampe UV-A avec un spectre entré sur un maximum à 385 nm, de puissance 8 W fournie par la société Philips. Pour chaque épaisseur, les mesures ont été effectuées pour différentes positions de la mousse.
a) Mousses translucides (la lumière peut passer à travers les alvéoles et à travers les murs de la mousse elle-même) : les résultats sont résumés dans le tableau 10.

**Tableau 10**

| Mousse de polyuréthane (taille des alvéoles supérieure à 4800 µm) | | | | | | |
|---|---|---|---|---|---|---|
| Ref. des mousses : Bulpren ™ S 32720 | | | | | | |
| Epaisseur [cm] | 0 | 1 | 2 | 1,46 | 3 | |
| Transmission [%] | 100 | 42.8 | 15.8 | 8.0 | 5.0 | |
| *Ces données peuvent être fittées par :* | | | | | | |
| *Transmission = exp(-épaisseur*/*1,106)* | | | | | | |
| *Le coefficient caractérise l'efficacité de transmission de la mousse* | | | | | | |

| Mousse de polyuréthane (taille moyenne des alvéoles: 2800 µm, diamètre 2300-3300µm) | | | | | | |
|---|---|---|---|---|---|---|
| Ref. des mousses : Bulpren™ S 28280 | | | | | | |
| Epaisseur [cm] | 0 | 1 | 2 | 3 | 4 | |
| Transmission [%] | 100 | 37.5 | 15.2 | 7.0 | 4.1% | |
| *Ces données peuvent être fittées par :* | | | | | | |
| *Transmission = exp(-épaisseur*/*0.977)* | | | | | | |

L'effet de la taille des alvéoles est assez modéré, car la lumière peut passer à travers les ponts de la mousse translucide.
b) Mousses non translucides (la lumière ne peut passer qu'à travers les alvéoles) : les résultats sont résumés dans le tableau 11 :

**Tableau 11**

| Mousse de polyuréthane (taille des alvéoles supérieure à 4500 µm) | | | | | | |
|---|---|---|---|---|---|---|
| Ref. des mousses : Bulpren™ S 32520 | | | | | | |
| Epaisseur [cm] | 0 | 1 | 2 | 3 | 4 | |
| Transmission [%] | 100 | 32.7 | 11.5 | 5.9 | 3.6 | |
| Ces *données peuvent être fittées par :* | | | | | | |
| *Transmission* = *exp(-épaisseur*/*0.8444)* | | | | | | |
| Mousse de polyuréthane (taille moyenne des alvéoles: 4500 µm, diamètre 3800 -5200 µm) | | | | | | |
| Ref. des mousses : Bulpren™ S 32450 | | | | | | |
| Epaisseur [cm] | 0 | 0,61 | 1,13 | 1,72 | 2,36 | |
| Transmission [%] | 100 | 24.1 | 7.0 | 3.8 | 3.1 | |
| Ces *données peuvent être fittées par :* | | | | | | |
| *Transmission* = *exp(-épaisseur*/*0.658)* | | | | | | |

On voit bien que l'influence de la taille des alvéoles sur la transmission de la lumière est plus importante dans le cas de mousses non translucides. Or, dès qu'une mousse est recouverte d'un photocatalyseur (par exemple de TiO₂), elle devient non translucide.

### Exemple 13 : Etude de l'influence de la taille des alvéoles sur les performances photocatalytiques.

On a utilisé le même réacteur que dans l'exemple 6. Cet exemple montre l'importance de la taille des alvéoles, des alvéoles de taille intermédiaire (cas 4) permettant d'obtenir des performances supérieures à celles obtenues sur mousses possédant des tailles d'alvéoles plus petites (cas 1) ou plus grandes (cas 2 et 3). Cet exemple montre aussi la supériorité des mousses testées dans l'exemple 4, de taille d'alvéoles interméidaires par rapport aux autres exemples.
Les résultats suivants (voir le tableau 12) ont été obtenus avec un photocatalyseur déposé sur mousse alvéolaire de PU selon l'invention.
Débit d'air sec : 0,4 L/min - Vitesse = 0.8 cm/s - Temps de séjour = 34 s
Concentration volumique en méthanol : 1200 ppm
TiO₂ : P25 de Degussa (Evonik)

**Tableau 12**

| | | | | | | |
|---|---|---|---|---|---|---|
| Cas 1 | Taille des alvéoles | Masse de TiO₂ [g] | 0 | 0,08 | 0,16 | 0,27 |
| | 2300-3300 µm (moyenne 2800 µm) Ref. des mousses : Bulpren™ S 28280 | cm de mousses | 0 | 2 | 4 | 6 |
| | | Conversion du méthanol [%] | 0 | 8 | 16 | 22 |
| | | | | | | |
| Cas 2 | Taille des alvéoles | Masse de TiO₂ [g] | 0 | 0,39 | 0.62 | |
| | Supérieure ou égale à | cm de mousses | 0 | 4 | 6 | |
| | 4500 µm Ref. des mousses : Bulpren™ S 32520 | Conversion du méthanol [%] | 0 | 28 | 38 | |
| | | | | | | |
| Cas 3 | Taille des alvéoles | Masse de TiO₂ [g] | 0 | 0,10 | 0,18 | 0,28 |
| | Supérieure ou égale à 4800 µm Ref. des mousses Bulpren ™ S 32720 | cm de mousses | 0 | 2 | 4 | 6 |
| | | Conversion du méthanol [%] | 0 | 20 | 35 | 54 |
| | | | | | | |
| Cas 4 | Taille des alvéoles | Masse de TiO₂ [g] | 0 | 0,09 | 0,18 | 0,28 |
| | 3800-5200 microns (moyenne 4500 microns) Ref. des mousses : Bulpren™ S 32450 | cm de mousses | 0 | 2 | 4 | 6 |
| | | Conversion du méthanol [%] | 0 | 31 | 44 | 65 |

### Exemple 14: Dépôt d'une couche de passivation sur une mousse alvéolaire de polyuréthane.

On disperse 4,11 g de AIO(OH) (nom commercial Disperal®, product Code 535100, fournisseur Akzo) dans 50 mL d'une solution aqueuse. La couche d'alumine a été obtenue par immersion de la mousse de PU dans la solution aqueuse de Disperal®, suivie d'un traitement thermique basse température, tel que par exemple 15 minutes à 200°C, ou bien même 15 minutes à 100°C.

Après la dernière couche d'alumine formée, on a réalisé le dépôt de TiO₂ P25, dispersé en solution aqueuse ou éthanol/eau :
- Préparation d'une suspension de TiO₂ dans H₂O à 100g/L ;
- Mise sous agitation vive pendant une nuit au moins (15h) ;
- Trempage de la mousse passivée ;
- Retrait de la mousse imprégnée ;
- Passage léger à l'air comprimé pour dégager l'obturation possible des alvéoles.
- Séchage à température ambiante

### Exemple 15: Préparation d'une mousse de PU passivée et dépôt de la phase photocatalytiquement active (hors invention).

Nous décrivons ici de manière schématique les étapes du procédé.
A) Dépôt et polymérisation d'un film inorganique de polysiloxane
   - Préparation du bain de polysiloxane par agitation magnétique pendant une dizaine de minutes ;
   - Arrêt de l'agitation ;
   - Trempage de la mousse macroscopique PU dans un bain de polysiloxane en prenant soin de bien chasser les bulles d'air prises dans le volume (secouer vigoureusement) afin d'imprégner tout le volume ;
   - Retrait de la mousse imprégnée ;
   - Egouttage primaire en agitant au dessus du bain (récupération de liquide) ;
   - Egouttage secondaire par dépôt des mousses imprégnées sur papier absorbant ;
   - Passage à l'air comprimé pour dégager l'obturation possible des alvéoles et égoutter efficacement pendant quelques secondes ;
   - Séchage à l'étuve à 150°C pendant 20 minutes (mousses déposées sur feuille de papier aluminium pour éviter attache) ;
   - Retrait des mousses de l'étuve ;
   - Repos à température ambiante 5 minutes ;
   - Renouvellement de l'opération pour cumul d'une seconde couche de polysiloxane.
B) Dépôt du photocatalyseur
   - Préparation d'une suspension de TiO₂ dans H₂O à 100g/L ;
   - Mise sous agitation vive pendant une nuit au moins (15h) ;
   - Trempage de la mousse préparée à l'étape A ;
   - Retrait de la mousse imprégnée ;
   - Passage léger à l'air comprimé pour dégager l'obturation possible des alvéoles ;
   - Séchage à température ambiante.

## Revendications

1. Photoréacteur comportant
- au moins un photocatalyseur comportant une mousse alvéolaire de carbone et une phase photocatalytiquement active, déposée directement sur ladite mousse alvéolaire, ou sur une phase intermédiaire déposée sur ladite mousse alvéolaire, la taille moyenne des alvéoles étant comprise entre 2500 µm et 5000 µm, préférentiellement entre 3000 µm et 5000 µm,
- un élément d'enveloppe étanche aux liquides et aux gaz,
- au moins une pièce dudit photocatalyseur étant située à l'intérieur dudit élément d'enveloppe, ladite au moins une pièce de photocatalyseur ayant une forme annulaire, et
- au moins une source d'un rayonnement lumineux,
**caractérisé en ce que**
(a) ledit photoréacteur comprend une pluralité de N pièces annulaires d'un photocatalyseur
(b) ledit rayonnement lumineux est introduit dans le diamètre intérieur desdites pièces annulaires,
(c) lesdites pièces annulaires présentent un diamètre intérieur alternativement différent, de manière à ce que toutes les pièces de numéro d'ordre pair présentent le même diamètre intérieur d₁, et toutes les pièces de numéro d'ordre impair présentent le même diamètre intérieur d₂.

2. Photoréacteur selon la revendication 1, **caractérisé en ce que** lesdites pièces annulaires sont séparées d'un espace vide ou d'une pièce optiquement transparente à au moins une partie dudit rayonnement lumineux utilisé.

3. Photoéacteur selon la revendication 1 ou 2, **caractérisé en ce que** ladite mousse alvéolaire de carbone présente une densité comprise entre 0,1 g/cm³ et 0,4 g/cm³.

4. Photoréacteur selon l'une quelconque des revendications 1 à 3, caractérisè en ce que ladite phase photocatalytiquement active est sélectionné dans le groupe constitué par :
- Les oxydes métalliques tels que WO₃, ZnO, TiO₂ et SnO₂, éventuellement dopés ou sur lesquels ont été greffés des éléments de transfert de charge,
- les titanates, éventuellement dopés,
- les sulfures ou séléniures métalliques, éventuellement dopés, tels que CdS, CdSe, ZnS, ZnSe et WS₂,
- les semiconducteurs de type III-V, éventuellement dopés, tels que GaAs et GaP.

5. Utilisation d'un photoréacteur selon l'une quelconque des revendications 1 à 4 pour catalyser des réactions chimiques en phase liquide.

6. Utilisation d'un photoréacteur selon l'une des revendications 1 à 4 pour l'inactivation ou dégradation d'agents biologiques.

## Patentansprüche

1. Photoreaktor, der Folgendes umfasst:
mindestens einen Photokatalysator, der einen Kohlenstoffzellschaum und eine photokatalytisch aktive Phase umfasst, die direkt auf dem besagten Zellschaum oder auf einer Zwischenphase abgelagert ist, die auf dem besagten Zellschaum abgelagert ist, wobei die durchschnittliche Größe von Zellen zwischen 2500 µm und 5000 µm, vorzugsweise zwischen 3000 µm und 5000 µm liegt,
- ein Umhüllungselement, das für Flüssigkeiten und Gas dicht ist,
- wobei mindestens ein Teil des besagten Photokatalysators sich im Inneren des besagten Umhüllungselements befindet, wobei das besagte mindestens eine Teil eine Ringform hat, und
- mindestens eine Quelle einer Lichtstrahlung,
**dadurch gekennzeichnet, dass**
(a) der besagte Photoreaktor eine Vielzahl von N ringförmigen Teilen eines Photokatalysators umfasst,
(b) die besagte Lichtstrahlung in den Innendurchmesser der besagten ringförmigen Teile eingeführt wird,
(c) die besagten ringförmigen Teile einen abwechselnd unterschiedlichen Innendurchmesser aufweisen, so dass alle Teile mit einer geraden laufenden Nummer denselben Innendurchmesser d₁ aufweisen und alle Teile mit einer ungeraden laufenden Nummer denselben Durchmesser d₂ aufweisen

2. Photoreaktor nach Anspruch 1, **dadurch gekennzeichnet, dass** die besagten ringförmigen Teile durch einen Leerraum oder ein Teil, das für mindestens einen Teil der verwendeten besagten Lichtstrahlung optisch transparent ist, getrennt sind.

3. Photoreaktor nach Anspruch 1 oder 2, **dadurch gekennzeichnet, dass** der besagte Kohlenstoffzellschaum eine Dichte zwischen 0,1 g/cm³ und 0,4 g/cm³ aufweist

4. Photoreaktor nach einem der Ansprüche 1 bis 3, **dadurch gekennzeichnet, dass** die besagte photokatalytisch aktive Phase aus der Gruppe ausgewählt ist, die aus Folgendem besteht:
- Metalloxiden, wie WO₃, ZnO, TiO₂ und SnO₂, die gegebenenfalls dotiert sind oder auf die Ladungstransferelemente gepfropft wurden,
- Titanaten, die gegebenenfalls dotiert sind, Metallsulfiden oder -seleniden, die gegebenenfalls dotiert sind, wie CdS, CdSe, ZnS, ZnSe und WS₂,
- Halbleitern vom Typ III-V, die gegebenenfalls dotiert sind, wie GaAs und GaP

5. Verwendung eines Photoreaktors nach einem der Ansprüche 1 bis 4 zum Katalysieren von chemischen Reaktionen in flüssiger Phase.

6. Verwendung eines Photoreaktors nach einem der Ansprüche 1 bis 4 zum Deaktivieren oder Abbauen von biologischen Agenzien.

## Claims

1. Photoreactor comprising
- at least one photocatalyst comprising a carbon alveolar foam and a photocatalytically active phase, deposited directly on said alveolar foam or on an intermediate phase deposited on said alveolar foam, the mean cell size being between 2500 µm and 5000 µm, preferentially between 3000 µm and 5000 µm,
- a shell element that is tight to liquids and gases,
- at least one piece of said photocatalyst being situated inside said shell element, said at least one piece of photocatalyst having an annular shape, and
- at least one luminous radiation source,
**characterised in that**
(a) said photoreactor comprises a plurality of N annular pieces of a photocatalyst,
(b) said luminous radiation is introduced into the internal diameter of said annular pieces,
(c) said annular pieces have an alternatively different internal diameter, such that all the pieces having an even sequential number have the same internal diameter d₁, and all the pieces having an odd sequential number have the same internal diameter d₂.

2. Photoreactor according to claim 1, **characterised in that** said annular pieces are separated from an empty space or an optically transparent piece to at least at portion of said luminous radiation used.

3. Photoreactor according to claim 1 or 2 **characterised in that** said carbon alveolar foam has a density between 0.1 g/cm³ and 0.4 g/cm³.

4. Photoreactor according to any of claims 1 to 3, **characterised in that** said photocatalytically active phase is selected in the group consisting of:
- Metal oxides such as WO₃, ZnO, TiO₂ and SnO₂, optionally doped or whereon load transfer elements have been grafted,
- titanates, optionally doped, metal sulphides or selenides, optionally doped, such as CdS, CdSe, ZnS, ZnSe and WS₂,
- type III-V semiconductors, optionally doped, such as GaAs and GaP

5. Use of a photoreactor according to any one of claims 1 to 4 for catalysing liquid-phase chemical reactions.

6. Use of a photoreactor according to any one of claims 1 to 4 for the inactivation or degradation of biological agents.
